# EUROPEAN PATENT SPECIFICATION

(11) **EP 1 290 201 B1**
(45) Date of publication and mention of the grant of the patent: **13.12.2006**
(21) Application number: 01944791.1
(22) Date of filing: 05.06.2001
(51) Int. Cl.: C12N 15/82, A61K 39/23, C07K 14/015

(54) **RECOMBINANT SUBUNIT PROTEINS FROM PORCINE PARVOVIRUS PRODUCED IN PLANTS**
IN PFLANZEN ERZEUGTE REKOMBINANTE PROTEINUNTEREINHEIT EINES PARVOVIRUS VOM SCHWEIN
SOUS-UNITES DE PROTEINES RECOMBINANTES PRODUITES DANS DES PLANTES A PARTIR D'UN PARVOVIRUS PORCIN

(30) Priority: 05.06.2000 US 208941 P
(43) Date of publication of application: 12.03.2003
(73) Proprietor: Her Majesty The Queen in Right of Canada as represented by The Minister of Agriculture and Agri-Food, London, ON N5V 4T3 (CA); Vaccine & Infectious Disease Organization, Saskatoon Saskatchewan, S7N 0W0 (CA)
(72) Inventor: RYMERSON, Robert, Her Majesty the Queen of Canada, Ontario N5V 4T3 (CA); BRANDLE, Jim, Her Majesty the Queen of Canada, OntarioN5V 4T3 (CA); BABIUK, Lorne, Veterinary Infectious Disease Org., Saskatoon, Saskatchewan S7N 5E3 (CA)
(74) Representative: Baldock, Sharon Claire
(86) International application number: PCT/CA2001/000818
(87) International publication number: WO 2001/094392

(56) References cited:
- EP-A- 0 551 449
- WO-A-94/20135
- TUBOLY T ET AL: "Immunogenicity of porcine transmissible gastroenteritis virus spike protein expressed in plants" VACCINE, BUTTERWORTH SCIENTIFIC. GUILDFORD, GB, vol. 18, no. 19, April 2000 (2000-04), pages 2023-2028, XP004202498 ISSN: 0264-410X

## Description

The present invention relates to the production of recombinant viral proteins in plants, and specifically to expression of porcine parvovirus VP2 subunit proteins in plants.

### BACKGROUND OF THE INVENTION

Porcine parvovirus is an autonomous replicating virus containing a single stranded DNA molecule of about 5100 nucleotides (Molitor *et al.,* 1984). Only the minus strand of the DNA is packaged into virions. The genome of the virus encodes three capsid proteins (VP1, VP2, VP3) and one non-structural protein (NS1). The capsid of parvovirus is about 22-25 nanometers in diameter and is comprised of VP1 and VP2 subunits. These proteins are derived from alternatively spliced versions of the same RNA molecule and thus overlap in sequence. Further, porcine parvovirus exhibits a high level of sequence similarity to feline panleukopenia virus, canine parvoviruses and rodent parvovirus (*Ranz et al.,* 1989). Porcine parvovirus has been linked to fetal death and mummification, reproductive failure in sows, diarrhea in young piglets, and a delayed return to oestrus (Dunne *et al* 1965; Joo and Johnson, 1976; Mengeling, 1978). Also, certain isolates or strains of the virus may cause skin lesions in pigs.

Serological studies show that porcine parvovirus is widespread in all swine-producing regions of the world with up to 80 % of animals showing seroconversion. Although there are differences in porcine parvovirus strains, some being extremely pathogenic and others being less pathogenic or totally non-pathogenic, when the virus becomes established or endemic in a country, the pathogenic strains appear to circulate in the population.

VP2 proteins are known to self-assemble into virus-like particles (VLPs) when produced in a baculovirus expression system (Martinez *et al.,* 1992). The VLPs have high immunogenic activity and are able to elicit protective immunity when administered systemically to pigs (Martinez *et al.,* 1992 and EP 0 551 449). However, the production of protein in baculovirus expression systems is complex and must be performed in a laboratory or similar setting. Further, the protein must be purified from baculovirus. The purification of protein from baculovirus increases the cost of protein production.

Molecular farming offers the potential for low-cost production of large amounts of protein that closely resembles that of the original host organism in both conformation and biological activity. Several proteins have been produced in plants including γ-interferon in tobacco (U.S. Pat No. 5,625,175), norwalk virus capsid protein in transgenic tobacco and potato plants (Mason *et al.* 1996), B subunit toxin of *Vibrio cholerae* in potato plants (*Arakawa et al.* 1998), and antigenic peptides from both rabies virus and mink enteritis virus in alfalfa. WO9420135 describes the production of plants expressing proteins of animal pathogens, to be used, inter alia, as edible vaccines.

Expressing transgenic proteins in plants offers many advantages over expressing transgenic proteins in other organisms such as bacteria. First, plants are higher eukaryotic organisms and thus have the same or similar intracellular machinery and mechanisms which govern protein folding, assembly and glycosylation as do mammalian systems. Further, unlike fermentation-based bacterial and mammalian cell systems, protein production in plants is not restricted by physical facilities. For example, agricultural scale production of recombinant proteins by plants is likely to be significantly greater than that produced by fermentation-based bacterial and mammalian cell systems. In addition, the costs of producing recombinant proteins in plants may be 10 to 50-fold lower than conventional bacterial bioreactor systems (Kusnadi *et al.* 1997). Also, plant systems produce pathogen free recombinant proteins. Further, the ability to produce biologically-active recombinant proteins in edible plant tissues or extracts allows low-cost oral delivery of proteins such as antigens as feed additives, and potentially eliminates the need for expensive down-stream purification processes of the protein.

There is a need in the art for proteins which confer protection against porcine parvovirus and the diseases caused by porcine parvovirus. Further, there is a need in the art for proteins which confer protection against porcine parvovirus and the diseases caused by porcine parvovirus and which may be produced in large quantities in plants. Also, there is a need for transgenic plants that express an immuno-reactive antigen of the porcine parvovirus which may be produced in relatively large quantities and purified.

It is an object of the present invention to overcome drawbacks in the prior art.

The above object is met by a combination of the features of the main claims. The sub-claims disclose further advantageous embodiments of the invention.

### SUMMARY OF THE INVENTION

The present invention relates to the production of recombinant viral proteins in plants, and specifically to expression of porcine parvovirus VP2 subunit proteins in plants.

According to the present invention there is provided a method for producing a protein in a plant, comprising:
i) transforming the plant with a nucleotide sequence expresses a porcine parvovirus VP2 subunit protein (SEQ ID NO:1) or a fragment or a derivative thereof; and
ii) growing the transformed plant.

The present invention also relates to a chimeric construct necessary to carry out the above method, and a plant, plant seed, plant cell, or progeny produced therefrom comprising a nucleotide sequence that encodes a porcine parvovirus VP2 subunit protein (SEQ ID NO:1), or a fragment or a derivative thereof, produced by the above method, and their use in the preparation of a medicament for prevention of porcine parvovirus infection in a pig.

The present invention also relates to a method as described above which further comprises harvesting the plant and purifying the protein, which may be formulated into a dosage form and administered to an animal.

### BRIEF DESCRIPTION OF THE DRA WINGS

These and other features of the invention will become more apparent from the following description in which reference is made to the appended drawings wherein:
- **FIGURE 1**: shows the general structure of a binary transformation vector (pCAVP) useful for the introduction of a AMV-VP2 gene into plants.
- **FIGURE 2**: shows the DNA coding sequence of the AMV-VP2 gene (SEQ ID NO:1).
- **FIGURE 3**: illustrates detection of the VP2 coding region in plants by PCR using untransformed control DNA (81V9), pCAVP plasmid DNA (C) and putative transgenic plant DNA (1-13).
- **FIGURE 4**: shows Reverse transcriptase PCR (RT-PCR) analysis of transgeneic plants containing a AMV-VP2 transgene. Products of RT-PCR reactions were resolved on a 1% agarose gel, and the VP2-specific product was approximately 1 KB. The numbers indicate the VP2-containing plant, with "+" and "-" indicating the addition or omission of reverse transcriptase in the first strand reaction. "C" contained RNA isolated from an untransformed plant. "P" stands for the plasmid positive control for the PCR. "W" contained water in place of template.
- **FIGURE 5**: shows a transmission electronic micrograph of a VP2-expressing tobacco plant. Virus-like particles of approximately 25 nm in diameter that accumulate in the cytoplasm are indicated with an arrow. The bar represents 50 nm.
- **FIGURE 6**: shows western analysis of transgenic plants carrying a AMV-VP2 gene (lanes 1,2, 5, 7, 9, 11.13,and 14) and an untransformed control plant (81V9).
- **FIGURE 7**: shows the results of a serum neutralization assay using fetal swine testicle cells, porcine parvovirus, and serum from immunized mice. The mice in groups VP2-9-1 and VP-9-2 were immunized with plant extract from a VP2-expressing plant, while groups 81V9-1, 81V9-2 and 81V9-3 received plant extract from untransformed control plants.

### DESCRIPTION OF PREFERRED EMBODIMENT

The present invention relates to the production of recombinant viral proteins in plants, and specifically, to expression of porcine parvovirus VP2 subunit proteins in plants.

According to an aspect of an embodiment of the present invention, there is provided a method of producing a protein in a plant comprising i) transforming the plant with a nucleotide sequence which expresses a porcine parvovirus VP-2 subunit protein (SEQ ID NO:1), a fragment, or derivative thereof, and ii) growing the transformed plant. In another aspect of an embodiment, the transformed plant may be harvested and the protein partially or fully purified and then optionally formulated into a dosage form and administered to an animal. It is conceivable to harvest the plant and feed it to an animal, but in a preferred embodiment of the invention, the plant is harvested and an extract is obtained which may be administered to an animal to confer resistance to porcine parvovirus. In a further embodiment, the porcine parvovirus VP-2 protein, fragment, or derivative thereof, is partially or fully purified, reformulated into a dosage form to be used as a vaccine in an animal, specifically to induce a mucosal immune response that renders an animal immune to parvovirus penetration at a mucosal site.

The porcine parvovirus VP-2 protein, fragment, or derivative thereof may be recognized as foreign by the immune system of an animal and may be capable of eliciting an immune response in the animal such that the animal acquires immunity to subsequent challenges by a virus, or by a bacterium or other agent which comprises the porcine parvovirus VP2 protein or fragment or derivative thereof.

A "derivative" includes any substitution, deletion, or addition to the nucleotide or amino acid sequence of the porcine parvovirus VP2 subunit protein (hereinafter, VP2), for example, but not limited to, the AMV-VP2 chimeric construct, provided that the derivative thereof, maintains the property of conferring resistance to a porcine parvovirus infection. A "fragment" includes any derivative of the porcine parvovirus VP2 subunit protein where one or more amino acid residues have been deleted from the amino terminus, the carboxy terminus or both the amino terminus and the carboxy terminus of the full length protein, provided that the fragment retains the capacity of conferring resistance to a porcine parvovirus infection..

By the term 'conferring resistance' it is meant stimulation of the immune system of an animal in response to foreign matter, such as but not limited to a protein, such that the foreign matter is neutralized and degraded by the immune system of an animal during a subsequent challenge. Stimulation of the immune system may include but is not limited to an increase in the production of a specific antibody against an antigen or a general increase in the production of many antibodies, increased production of immune cells, such as but not limited to macrophages and lymphocytes, or increased production of immunomodulators such as but not limited to interferons and interleukins, or a combination thereof. In addition the immune response may occur at a specific site, for example, but not wishing to be limiting, a mucousal membrane which may be a site of entry of foreign matter.

The present invention provides an effective method for the reliable production of VP2 (or a fragment or derivative thereof). In order to optimize the expression of the foreign gene within plants, the native or synthetic gene may be used or altered as required so that the corresponding protein is produced at a level higher than the native gene. For example, which is not to be considered limiting, the gene may be a synthetic gene, optimized for codon usage within a plant, comprising at least about 80% homology with the native VP2 as determined using sequence comparison techniques for example but not limited to BLAST algorithm (GenBank: www.ncbi.nlm.nih.gov/cgi-bin/BLAST/), using default parameters (Program: blastn; Database: nr; Expect 10; filter: low complexity; Alignment: pairwise; Word size:11).

Analogs or derivatives thereof also include those sequences which hybridize under stringent hybridization conditions to the DNA sequence of SEQ ID NO: 1, provided that the sequences exhibit the property as described herein. The stringent hybridization conditions are described in Molecular Cloning (A Laboratory Manual), Cold Spring Harbor Laboratory, 1982, p. 387-389 and are: incubation for 12-16 hours in a hybridization solution of 6x SSC, 0.01M EDTA, 5x Denhart's solution, 0.5% SDS and 100 µg/ml denatured salmon sperm DNA at 68°C, followed by a 5 minute wash in a 2x SSC and 0.5% SDS solution at room temperature, followed by a 15 minute incubation at room temperature in a 2x SSC and 0.1% SDS solution, followed by a 2 hour incubation at 68°C in a 0.1 x SSC and 0.5% SDS solution and followed by a further 30 minute incubation at 68°C in a 0.1x SSC and 0.5% SDS solution.

It is also contemplated that fragments or portions of VP2 or derivatives thereof, that exhibit useful biological properties, for example, but not limited to antigenic properties, may be expressed within plant tissues. Preferably, fragments or portions of VP2, or derivatives thereof, exhibit properties that aid in the health and productivity of animals similar to those observed with the administration of native VP2.

Furthermore, other modifications of the native VP2 or synthetic VP2 (sVP2) gene may occur so that expression of the gene, and stability or purification of the protein can be optimized. For example, modification of the 5' or 3' region of these genes can be carried out in order to enhance expression of the gene and target the product to an appropriate intercellular compartment to ensure stability. An example of 5'modification may include a signal peptide (signal sequence) to direct the protein to a specific cellular compartment, for example which is not to be considered limiting in any manner, the signal sequence from a tobacco pathogenesis related (PR) protein (Cornelissen et al. 1986, Nature 321:531-532), or the alfalfa mosaic virus (AMV) leader sequence (Jobling and Gehrke, 1987, Nature 325:622-625). An example of a 3' modification includes a histidine tag which can be used to aid in purification of the encoded protein. However, other alterations to the 5', 3', regions, or internal modifications may be utilized in order to optimize expression, stability and, optionally, purification of the expressed protein. It is preferred that the synthetic gene sVP2, encoding the mature protein comprises a codon bias similar to highly expressed genes found in plants carry, and if desired, comprises a motif that allows for simple extraction, for example an affinity tag, such as but not limited to a His-tag as is known in the art. The affinity-tag of the protein may be used for the purification of the protein using chromatography, for example a Ni²⁺ column may be used for the purification of HIS-tag containing proteins. It is also contemplated that the protein may be modified so that the immature protein is targeted to a compartment of the cell to enhance stability of the product, for example the plastid, or the lumen of the endoplasmic reticulum (ER). However, other sites may also be targeted for example, extracellular secretion, in order to simplify extraction protocols, or the mitochondria compartment.

By the term "expression" is meant the production of a functional RNA, protein or both, from a gene or transgene.

By "gene", it is meant a particular sequence of nucleotides including the coding region, or fragment thereof, and optionally the promoter and terminator regions which regulates expression of the gene, as well as other sites required for gene expression for example a polyadenylation signal which regulates the termination of transcription. By "coding region" or "structural gene", it is meant any region of DNA that determines the primary structure of a polypeptide following genetic transcription and translation. Furthermore, fragments comprising regions of interest of a coding region or structural gene may also be employed as needed. By "transgene" is meant genes that have been introduced into a host cell or organism that would not normally be present in the host cell or organism, particularly nucleic acids that have been modified by recombinant DNA techniques. The term "transgene" also includes host genes that are placed under the control of a new promoter or terminator sequence, for example. By "synthetic gene" it is meant a DNA sequence of a structural gene that is synthesized using methods known in the art for example but not limited to chemical syntheses, site directed mutagenesis, or PCR and related techniques. A synthetic gene can comprise a fragment or the entire coding region of VP2. Furthermore, a synthetic gene may also comprise regulatory elements that enhance expression of the gene, or motifs that aid in the stability or cellular targeting of the protein product. It is also contemplated that a synthetic gene optionally includes regions useful for the isolation and purification of the protein, or the protein fragment, encoded by the synthetic gene such as an affinity-tag.

By "codon optimization" it is meant the selection of appropriate DNA nucleotides for the synthesis of oligonucleotide building blocks, and their subsequent enzymatic assembly, of a structural gene or fragment thereof in order to approach codon usage within plants.

In order to maximize expression levels and transgene protein production of VP2 or fragment or derivatives or fragments thereof, the nucleic acid sequence of VP2 is examined and the coding region modified to optimize for expression of the gene in plants, using a procedure similar to that outlined by Sardana et al. (Plant Cell Reports 15:677-6.81; 1996). A table of codon usage from highly expressed genes of dicotyledonous plants is compiled using the data of Murray et al. (Nuc Acids Res. 17:477-498; 1989). This information is used to compile a synthetic sequence for sVP2.

Assembly of the sVP2 gene of this invention is performed using standard technology know in the art. The gene may be assembled enzymatically, within a DNA vector, for example using PCR, or synthesised from chemically synthesized oligonucleotide duplex segments. The synthetic VP2 gene is then transformed to plant genomes using methods known in the art. Expression of the gene may be determined using methods known within the art, for example Northern analysis, or ELISA.

Therefore, the present invention contemplates a gene for VP2 optimized for expression in plants. The sVP2 is synthesized to contain a suitable signal sequence, for example the AMV leader sequence, and a 6 x histidine tail.

In an aspect of an embodiment, the.method of the present invention relates to transforming a plant with a chimeric construct which expresses a porcine parvovirus VP2 subunit protein, a fragment, or a derivative thereof in a plant. The chimeric construct comprises a nucleotide sequence which encodes a porcine parvovirus VP2 subunit protein, (SEQ ID NO:1), a fragment or a derivative thereof, in operative association with regulatory sequences active in plants. Any regulatory sequence may be used, for example, but not limited to, a constitutive sequence.

A constitutive sequence directs the expression of a gene throughout the various parts of a plant and continuously throughout plant development. Examples of known constitutive sequences include promoters associated with the CaMV 35S transcript. (Odell et al., 1985, *Nature,* **313:** 810-812), the rice actin 1 (Zhang et al, 1991, *Plant Cell,* **3**: 1155-1165) and triosephosphate isomerase 1 (Xu et al, 1994, *Plant Physiol.* **106**:459-467) genes, the maize ubiquitin 1 gene (Cornejo et al, 1993, *Plant Mol. Biol.* **29:** 637-646), the *Arabidopsis* ubiquitin 1 and 6 genes (Holtorf et al, 1995, *Plant Mol. Biol.* **29:** 637-646), and the tobacco translational initiation factor 4A gene (Mandel et al, 1995 *Plant Mol. Biol.* **29:** 995-1004). The term "constitutive" as used herein does not necessarily indicate that a gene under control of the constitutive sequence is expressed at the same level in all cell types, but that the gene is expressed in a wide range of cell types even though variation in abundance is often observed.

A regulatory sequence may also include, but is not limited to promoter elements, basal (core) promoter elements, elements that are inducible in response to an external stimulus, elements that mediate promoter activity such as negative regulatory sequences or.transcriptional enhancers. Regulatory sequences may also comprise elements that are active following transcription, for example, regulatory sequences that modulate gene expression such as translational and transcriptional enhancers, translational and transcriptional repressors, upstream activating sequences, and mRNA instability determinants. Several of these latter elements may be located proximal to the coding region. In the context of this disclosure, the regulatory sequence typically refers to a sequence of DNA, usually, but not always, upstream (5') to the coding sequence of a structural gene, which controls the expression of the coding region by providing the recognition for RNA polymerase and/or other factors required for transcription to start at a particular site. However, it is to be understood that other nucleotide-sequences, located within introns, or 3' of the sequence may also contribute to the regulation of expression of a coding region of interest. An example of a regulatory sequence that provides for the recognition for RNA polymerase or other transcriptional factors to ensure initiation at a particular site is a promoter sequence. A promoter sequence comprises a basal promoter sequence, responsible for the initiation of transcription, as well as other regulatory sequences (as listed above) that modify gene expression.

There are also several types of regulatory sequences, including those that are developmentally regulated, inducible and constitutive. A regulatory sequence that is developmentally regulated, or controls the differential expression of a gene under its control, is activated within certain organs or tissues of an organ at specific times during the development of that organ or tissue. However, some regulatory sequences that are developmentally regulated may preferentially be active within certain organs or tissues at specific developmental stages, they may also be active in a developmentally regulated manner, or at a basal level in other organs or tissues within the plant as well.

An inducible regulatory sequence is one that is capable of directly or indirectly activating transcription of one or more DNA sequences or genes in response to an inducer. In the absence of an inducer the DNA sequences or genes will not be transcribed. Typically the protein factor, that binds specifically to an inducible sequence to activate transcription, may be present in an inactive form which is then directly or indirectly converted to the active form by the inducer. However, the protein factor may also be absent. The inducer can be a chemical agent such as a protein, metabolite, growth regulator, herbicide or phenolic compound or a physiological stress imposed directly by heat, cold, salt, or toxic elements or indirectly through the action of a pathogen or disease agent such as a virus. A plant cell containing an inducible sequence may be exposed to an inducer by externally applying the inducer to the cell or plant such as by spraying, watering, heating or similar methods. Inducible elements may be derived from either plant or non-plant genes (e.g. Gatz, C. and Lenk, I.R.P., 1998, Trends Plant Sci. 3, 352-358; which is incorporated by reference). Examples, of potential inducible promoters include, but not limited to, teracycline-inducible promoter (Gatz, C.,1997, Ann. Rev. Plant Physiol. Plant Mol. Biol. 48, 89-108; which is incorporated by reference), steroid inducible promoter (Aoyama, T. and Chua, N.H.,1997, Plant J. 2, 397-404; which is incorporated by reference) and ethanol-inducible promoter (Salter, M.G., et al, 1998, Plant Journal. 16, 127-132; Caddick, M.X., et al,1998, Nature Biotech. 16, 177-180, which are incorporated by reference) cytokinin inducible *IB6* and *CKI1* genes (Brandstatter, I. and Kieber, J.J.,1998, Plant Cell 10, 1009-1019; Kakimoto, T., 1996, Science 274, 982-985; which are incorporated by reference) and the auxin inducible element, DR5 (Ulmasov, T., et al., 1997, Plant Cell 9, 1963-1971; which is incorporated by reference).

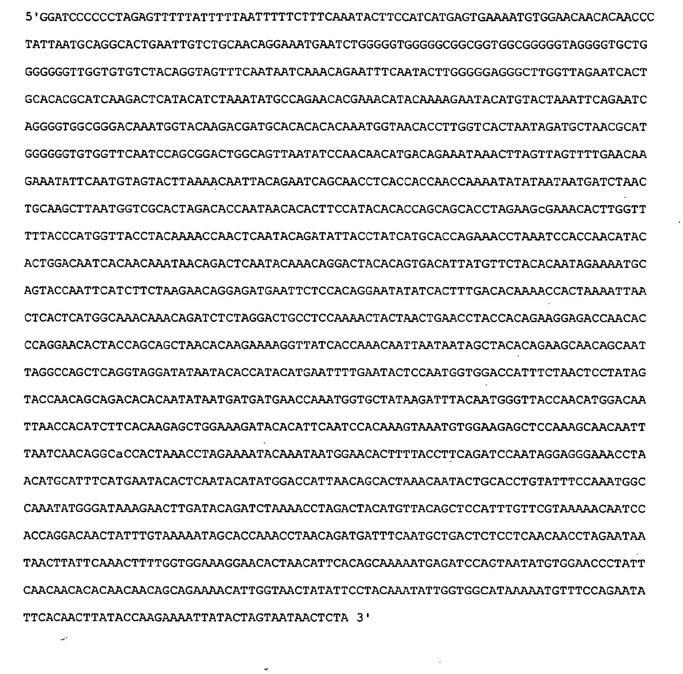

The nucleotide sequence used in the method of the present invention thus includes the DNA sequences of SEQ ID NO: 1 (see also Figure 2), and fragments or derivative thereof, as well as analogues of, or nucleic acid sequences comprising about 80% similarity with the nucleic acids as defined in SEQ ID NO: 1. Analogues include those DNA sequences which hybridize under stringent hybridization conditions (described above, see Maniatis *et al.,* in Molecular Cloning (A Laboratory Manual), Cold Spring Harbor Laboratory, 1982, p. 387-389) to the DNA sequence of SEQ ID NO: 1, provided that said sequences maintain at least one property of the activity of the gene as defined herein.

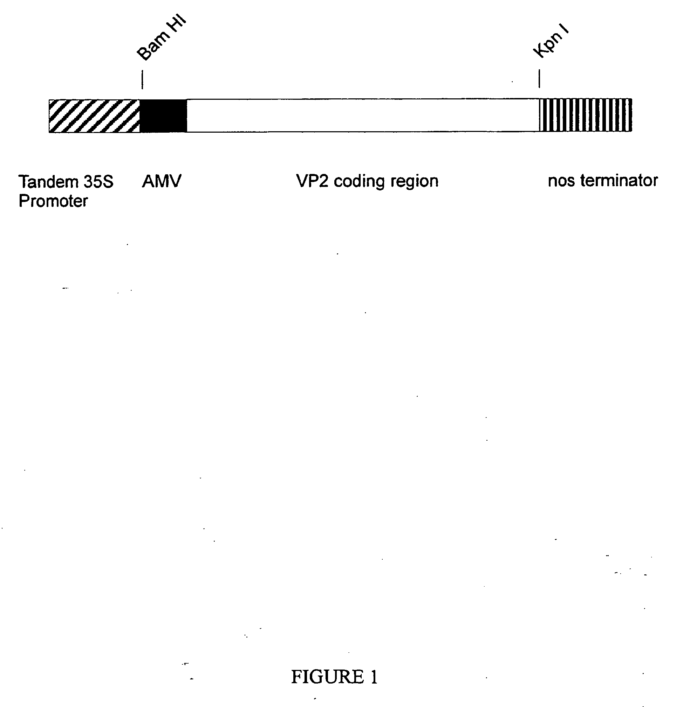

In an embodiment of the method of the present invention, the coding region of the VP2 subunit protein may be linked to as part, for example, but not limited to the alfalfa mosaic virus leader sequence and the fused sequence may be cloned into a vector suitable for expression in a plant, for example, but not limited to a pCAVP binary vector (Figure 1) comprising a desired regulatory region, for example, but not limited to a tandem 35S CaMV promoter and a nos terminator. The pCAVP binary vector comprising the cloned AMV-VP2 gene may be introduced into a transforming strain, for example, but not limited to an *Agrobacterium tumefaciens* strain containing a disarmed Ti plasmid, and plants may be transformed using methods described in the art. However, as one of skill in the art will understand, there exists many other vectors, promoters, terminators and transformation systems which may be used in place of those described herein. For example, but not wishing to be limiting, the constructs of the present invention can be introduced into plant cells using Ti plasmids, Ri plasmids, plant virus vectors, direct DNA transformation, micro-injection, electroporation, etc. For reviews of such techniques see for example Weissbach and Weissbach, *Methods for Plant Molecular Biology,* Academy Press, New York VIII, pp. 421-463 (1988); Geierson and Corey, *Plant Molecular Biology,* 2d Ed. (1988); and Miki and Iyer, Fundamentals of Gene Transfer in Plants. In *Plant Metabolism,* 2d Ed. DT. Dennis, DH Turpin, DD Lefebrve, DB Layzell (eds), Addison Wesly, Langmans Ltd. London, pp. 561-579 (1997). The present invention further includes a suitable vector comprising the chimeric gene construct.

To aid in identification of transformed plant cells, the constructs of this invention may be further manipulated to include plant selectable markers. Useful selectable markers include enzymes which provide for resistance to chemicals such as an antibiotic for example, gentamycin, hygromycin, kanamycin, or herbicides such as phosphinothrycin, glyphosate, chlorosulfuron, and the like. Similarly, enzymes providing for production of a compound identifiable by colour change such as GUS (β-glucuronidase), or luminescence, such as luciferase or GFP, are useful.

The method of the present invention may be practiced in any plant including forage plants, food crops or other plants depending on the need. For example, but not wishing to be limiting, the present invention may be practiced in tobacco, com, barley, alfalfa and potato. Preferably, the method of the present invention is practiced in low nicotine tobacco plants.

The present invention includes plants, plant cells or plant seeds comprising a nucleotide sequence which encodes a porcine parvovirus VP2 subunit protein (SEQ ID NO:1), a fragment or a derivative thereof.

The protein produced by the method of the present invention may comprise full-length porcine parvovirus VP2 subunit protein or a fragment or derivative thereof. As will be appreciated by someone of skill in the art, an entire protein may not be required to immunize an animal to subsequent challenges by the protein or an infectious agent comprising the protein, but rather, it may be possible that a smaller fragment of the protein can immunize an animal against subsequent challenges by the full-length protein, or an infectious agent comprising the full-length protein, such as but not limited to a bacterium or a virus. Further, as someone of skill in the art will understand, viruses often undergo mutations in genes which permit the virus to escape immune detection in an animal. Thus, the method of the present invention also contemplates that different porcine parvovirus strains may have different VP2 subunit protein sequences and therefore natural variants or man-made variants of the porcine parvovirus VP2 subunit protein, or fragment or derivative thereof, are fully contemplated to be included by the present invention.

According to another aspect of the invention, the transgenic plants expressing the porcine parvovirus VP2 subunit protein (SEQ ID NO:1), as well as plant seeds, plant cell or progeny produced therefrom, can be used in the manufacture of a medicament for the prevention of porcine parvovirus infection in an animal, specifically a pig.

For this purpose, it is conceivable that the plant itself may be added directly to the chow that is consumed by the pig. The plant, or plant tissue, may be harvested and fed directly to the animal, or the harvested tissue may be dried prior to feeding, or the animal may be permitted to graze on the plant without prior harvesting taking place. The harvested plant tissues could be used as a food supplement within animal feed. If the plant tissue is being fed to an animal with little or no further processing, the plant tissue should be edible.

According to a preferred embodiment, the VP2 protein produced by plants of the present invention is either contained in an extract of the plant, or is partially or completely purified and reformulated into a desired dosage form. The dosage forms may comprise an oral dosage form wherein the protein is dissolved in a suitable excipient, for instance water.

In addition, the protein may be formulated into an injectable dosage form. An injectable dosage form may include other adjuvants that may function to enhance the immunogenicity of the protein. For example, but not meant to be limiting, the protein or partially purified protein may be combined with Freund's adjuvant. Thus, the protein produced by the method of the present invention confers immunity against parvovirus in animals that have not been previously challenged with the virus, when the same animals are injected with a dosage form of a protein composition comprising the partially or completely purified protein produced by the method of the present invention. For example, but not meant to be limiting, when mice are injected with a dosage form of a plant extract comprising the protein produced by the method of the present invention an immune response is elicited, such that serum collected from the injected mice is capable of conferring resistance against porcine parvovirus in an *in vitro* assay.

Such medicaments may be used to confer immunity to porcine parvovirus in pigs that have not been previously challenged with the virus, regardless of whether the protein produced by the method of the present invention contained full-length VP2 subunit protein, a fragment or a derivative thereof.

The immunity may be induced at mucus membranes such as nasal mucus membranes and gastrointestinal mucus membranes. Inducing immunity at mucus membranes may permit neutralization of the virus at the site of entry of the animal. Further, serum antibody isotopes may be assayed to determine whether killed VLPs can stimulate immune cells, such as but not limited to macrophages, helper T-cells, cytotoxic T-cells, neutrophils, or combinations thereof. In addition, the presence of cytokines such as but not limited to interferons, and interleukins may be assessed. Results suggest that there may be a correlation between the levels of cytokines, the levels of immune cells or the presence of serum antibodies in animals treated with the protein produced by the method of the present invention.

Following immunization, pregnant sows may be challenged, without wishing to be limiting, orally, and the level of protection may be determined. The level of protection may be assessed by monitoring clinical signs and assessing the ability of the virus to cross the placenta and infect the fetus. In the fetus, the presence of viral antigens can be assayed by immunohistochemical studies and by virus isolation.

The protein produced by the method of the present invention, which comprises viral recombinant VP2 sub-unit proteins and fragments thereof, may have a variety of uses including, but not limited to the production of immunogenically active sub-unit proteins for use as oral proteins, the production of immunogenically active sub-unit proteins for the use as systemically administered proteins, the production of antibodies for veterinary diagnostic use, for general research purposes or combinations thereof. Further, the protein produced by the method of the present invention may be produced in large quantities in plants, isolated and optionally purified at potentially reduced costs compared to other conventional methods of producing proteins such as but not limited those which employ fermentation processes. It is also contemplated that the VP2 protein, fragments or derivatives thereof may be used to carry epitopes from other anitgens. Such a hybrid protein may also be used to elicit protective immunity to the animal.

The present invention will be further illustrated in the following examples. However, it is to be understood that these examples are for illustrative purposes only, and should not be used to limit the scope of the present invention in any manner.

### Example 1: Construction of VP2 Plant Expression Vectors

The alfalfa mosaic virus (AMV) leader sequence (Jobling and Gehrke, 1987) is added to the VP2 gene by PCR (Figure 1). A 70 nucleotide primer (AMVVP; SEQ ID NO:2):
5'GGGTCTAGAGTTTTTATTTTTAATTTTTCTTTCAAA
TACTTCCATCATGAGTGAAAATGTGGAACAACAC -3'
is synthesized. PCR amplification is performed using 5.25 units of Expand DNA polymerase (Boehringer Mannheim), 350 µmol dNTPs, 1.75 mM MgCl₂, 10 pmole of AMVVP and T7 primers and 5 ng of template (pBluescript KS+ containing the VP2 gene). Thirty PCR cycles are performed according to the manufacturer's instructions, using an annealing temperature of 65 °C. The amplified DNA fragment is purified using standard methods in the art, end filled, and introduced into the Sma I restriction site of pBluescript KS+. The resulting plasmid (pBSAVP) is digested with BamHI and KpnI restriction enzymes and the AMV-VP2 fragment, shown in Figure 2, is introduced into the binary vector pCamter X which was previously digested with the same enzymes. This binary vector contains a duplicated 35S promoter (Kay *et al* 1987), a multiple cloning region, the nos terminator, and further comprises neomycin phosphotransferase II (npt II) as the selectable marker between the T-DNA border sequences. Similar results may be obtained using the tCUP promoter (Miki *et al.* 1998).

### Example 2: Plant Transformation with the VP2 Expression Vector

The pCAVP binary vector is introduced into *Agrobacterium tumefaciens* (EHA 105 which contains the disarmed Ti plasmid pEHA 104) using standard protocols within the art (*Horsch et al.* 1985). Low nicotine tobacco plants from the cultivar 81-V9 are grown aseptically for transformation. The midvein of the leaf material is excised and the leaf material is cut into 1 cm² fragments using a scalpel. The fragments are precultured with the epidermal side down for 2 days on solid media consisting of MS (Murishage and Skoog) salts, B5 vitamins, 3% sucrose, 1 mg/L 6-benzylaminopurine (BA) and 0.1 mg/L - α-naphthalene acetic acid (NAA).

Infection of leaf explants with transgenic *Agrobacterium* from cultures grown overnight in LB (Luria Broth) medium is achieved by submerging leaf explants in the agrobacterium cultures that were diluted about 50 %, followed by blotting on Whatman No.2 filter paper to remove excess bacteria. The explants are returned to the solid medium described above and allowed to grow for 2 days. Subsequently, bacterial growth was inhibited and selection for transformants was initiated by transferring explants to medium containing 500 µg/mL Timentin and 100 µg/mL kanamycin. The explants were transferred to fresh media every 2- 3 weeks until shoots emerged.

After the explants develop well-defined stems, the shoots are excised and transferred to Magenta boxes containing solid media. The solid media comprises MS salts, B5 vitamins, 3% sucrose, 500 µg/ml Timentin and 100 µg/ml kanamycin. Roots are allowed to develop from the putative transgenic plants at which point the plants are placed in soil-less potting mix in a 15 cm plastic pot and transferred to the greenhouse. To ensure that each plant has arisen from an independent transformation event the explants are divided into separate fragments early in the procedure and only 1 shoot was selected from each fragment.

### Example 3: Analysis of the AMV-VP2 Insertions in Transgenic Plants

Each of the putative transgenic tobacco plants resulting from the agrobacterium transformation is screened for the presence of the VP2 gene using PCR. The primers chosen for PCR amplification are VP1060F:
GGTGGACCATTTCTAACTC (SEQ ID NO:3)
   and nos:
CCGGCAACAGGATTCAATCTTAA (SEQ ID NO:4).

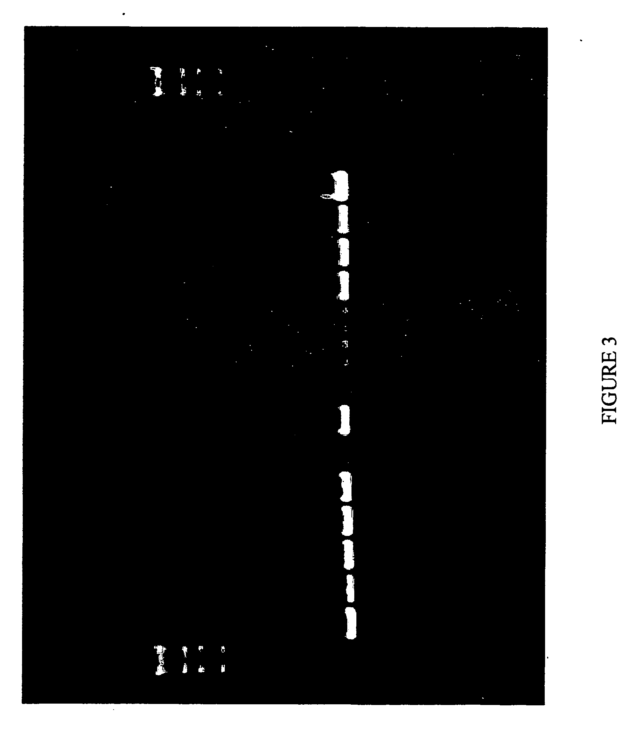

DNA is extracted from plant tissue using standard methods known in the art and is subjected to 35 cycles of amplification using 10 pMol of both primers, 100 mM dNTPs, 2 mM MgCl₂, 1 U Taq polymerase and approximately 50 ng of plant DNA. Thirteen plants were confirmed positive by PCR, as shown in Figure 3.

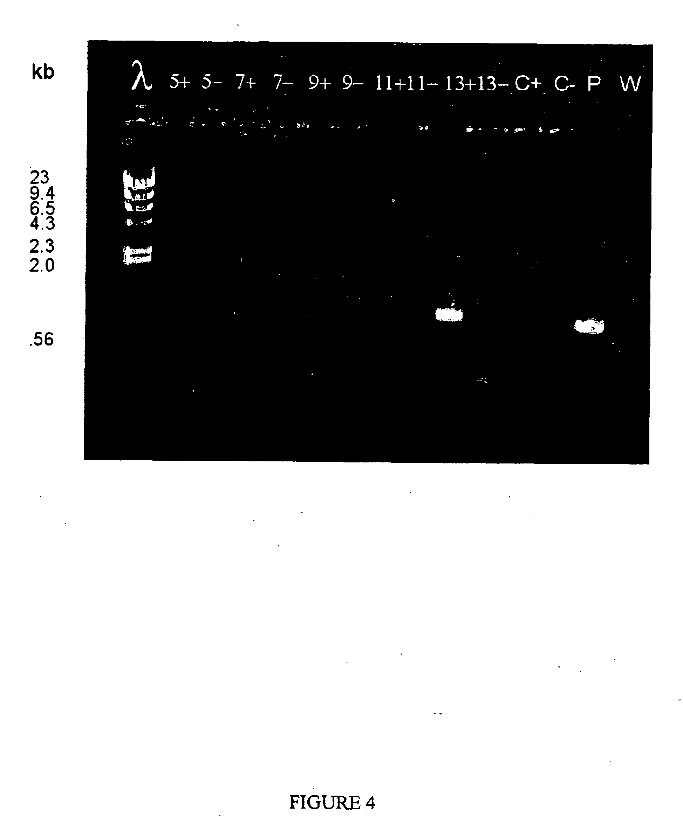

Transgenic tobacco plants are assayed for expression of VP2 RNA by RT-PCR For RT-PCR, 10 micrograms of RNA was treated with RNase-free DNase and 5 ug of this was used for cDNA synthesis. Oligo dT (12-18) (0.05 ug) was mixed with the RNA incubated at 70°C, and chilled on ice. First strand synthesis proceeded for 50 minutes at 42°C in a buffer containing 10 mM DTT. 0.5 mM dNTP, 50 mM Tris HCl pH 8.3, 75 mM KCl, 3 mM MgCl₂, and 200 units of Superscript II reverse transcriptase (Gibco BRL Life Technologies). The enzyme was heat-inactivated at 70°C for 15 minutes. As a negative control, reverse transcription reactions that did not contain any reverse transcriptase were done for each sample. PCR was then done as described above with 2 uL of the reverse transcription reaction as the template and primers specific to VP2. The synthesis of the first strand was confirmed by testing it as a template for PCR with primers for the actin gene. Transcript for VP2 was detected in five of the plants tested as shown in Figure 4.

### Example 4: Detection of the VP2 Protein in Transgenic Plants

### Transmission electron microscopy

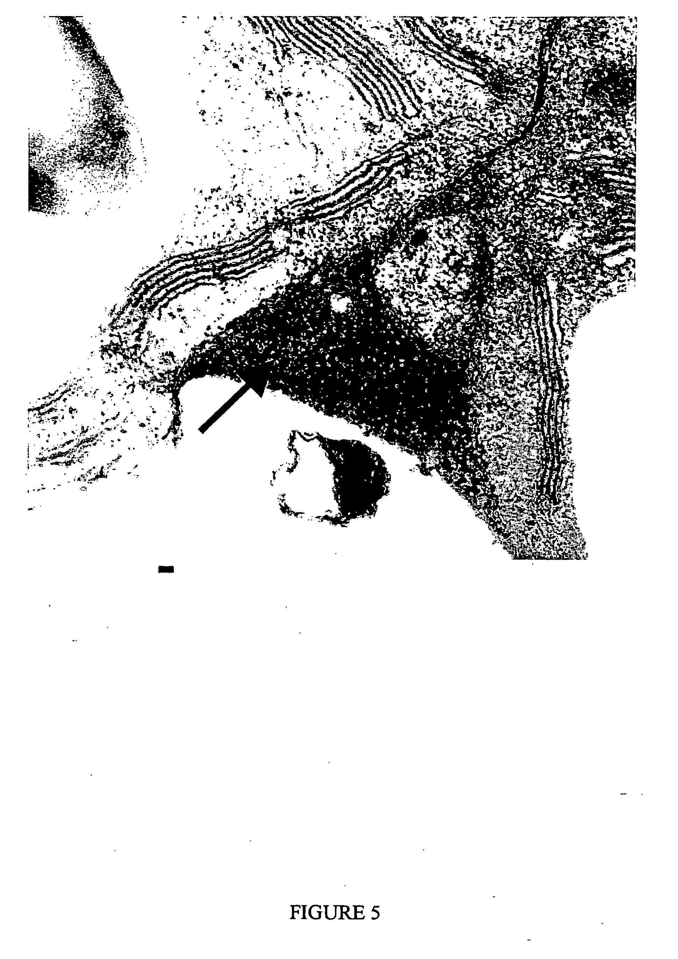

Tissue samples of one transgenic plant, CAVP12-9, which expresses VP2 were analysed by transmission electron microscopy. They were embedded in plastic and sectioned. Structures of approximately 25 nm in diameter, consistent with the size and shape of virus-like particles, were observed in the cytoplasm of the plant cells, as shown in Figure 5.

### Immunodetection of the VP2 protein in plant extracts

Soluble protein is extracted from 2g of leaf tissue from each transgenic plant. The tissue is ground to a paste in a chilled mortar and pestle with a small amount of acid-washed sand as an abrasive. 4 mL of extraction buffer comprising 50 mM Tris HCl pH 8.0, 5% glycerol, 0.1 M KCI, 2 mM EDTA, 2 µg/mL, leupeptin, pepstatin and 1.5% polyvinylpolypyrrolidone, is added and the tissue is ground to into a homogenate. The homogenate is filtered through Miracloth^{™} to remove large particles and then centrifuged at 10,000 x g at 4°C for 20 min. The supernatant is removed, divided into small aliquots and frozen at - 20°C until needed.

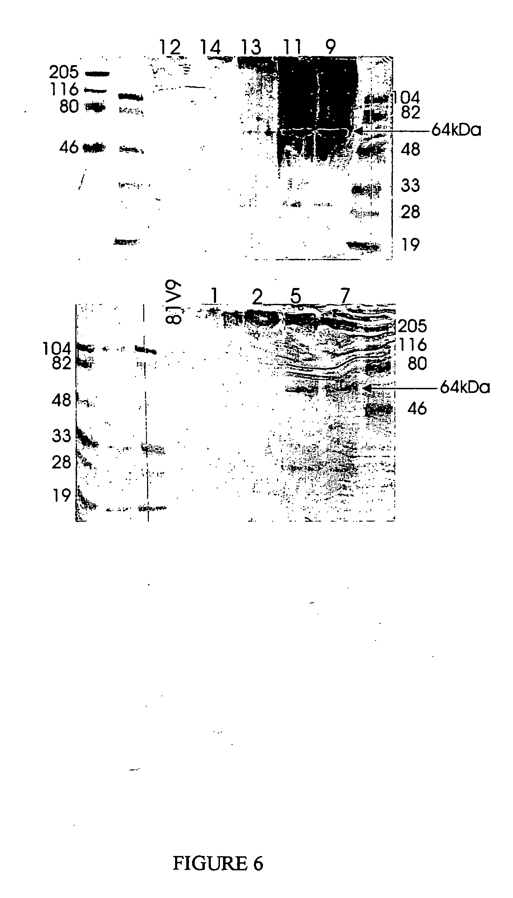

Western blot analysis is performed on the protein extracts using a commercially available monoclonal antibody capable of binding VP2 protein using standard methods. Proteins were separated by SDS-PAGE on a 10 % gel, and transferred onto nitrocellulose membrane. The membrane is blocked for 45 minutes at room temperature in TBS containing 10 % bovine serum albumin (BSA), then washed once for 5 minutes and then twice for 15 minutes in TBST containing 5 % skim milk and incubated with a primary antibody directed against VP2 protein (1/20 dilution). Following an overnight incubation, the membrane is washed, incubated with a secondary antibody (1/1000 dilution) for 45 minutes and then washed 3 times each for 5 minutes in TBST. Following developing of the blot, a protein of approximately 64kDa cross reacting with the VP2 specific antibody was detected in four of the transgenic plants, as shown in Figure 6.

### Example 5: Small Animal Trials

### Small animal trials by injection of protein composition comprising VP2

Seed collected from primary transformants was plated on MS media containing 300mg/mL kanamycin and was allowed to germinate and grow for three weeks. From the germinated seed, green transgenic seedlings were planted in soil-less potting mix and grown for approximately six weeks in a greenhouse. Leaf tissue was then collected and flash frozen in liquid nitrogen, or used fresh. Leaves were coarsely chopped with a razor blade and ground to a pulp with two volumes of buffer (50 mM Tris-HCl, pH 8.0; 0.1 M KCl; 1 mM EDTA; 15 mg/mL PVPP; 5% glycerol) in a pre-chilled Waring blendor. The homogenate was strained through one layer of Miracloth to remove large particles and then centrifuged (8,000 x g, 20 min, 4 °C). The supernatant was decanted and centrifuged again using the same conditions. The final supernatant was divided into aliquots and frozen. The same procedure was repeated with untransformed 81V9 plant tissue.

The crude protein extracts were thawed, centrifuged, and filtered through a 0.45 um filter. Groups of 3 to 4 Swiss mice were injected with plant extract. The control group received 125 ug plant extract from untransformed plants per mouse, while the experimental group received 450 ug plant extract from plant CAVP12-9 per mouse. The protein extracts were mixed with equal volumes of Freund's complete adjuvant for the first injection to a total volume of 100 uL, and with Freund's incomplete adjuvant for the second and third injections. Subcutaneous injections were done on days 0, 21 and 42 into the hind legs of the mice, with half of the volume being injected into each leg. Blood was collected before the first injection on day 0, and on day 52.

The serum neutralization assay was done using fetal swine testicle cells (ATCC #CRL 1746). Swine testicle cells were grown for 18 hours at 37°C, 5 % CO₂ in MEM in a 48 well tissue culture plate. Mouse serum from the control and experimental mice was heat-inactivated for 30 minutes at 56 ° C. Serial dilutions of the mouse serum were done in MEM plus two percent fetal bovine serum at 1/300, 1/900, 1/2700, and 1/8100. Porcine parvovirus was diluted to give 50 plaque-forming units/ml. Equal volumes of the diluted PPV and the dilutions of the mouse sera were mixed and incubated for 1.5 hours at 37 °C. The PPV plus sera mixtures were then added to the wells of the confluent plate of swine testicle cells (400 uL/well, in duplicate) and incubated at 37 °C for 18 hours. The volume in the wells was increased to 1 ml with MEM and incubation was continued for 4 to 5 days. The number of plaques was counted and the neutralization titre was determined by calculating the dilution which reduced the plaque number by 50 percent.

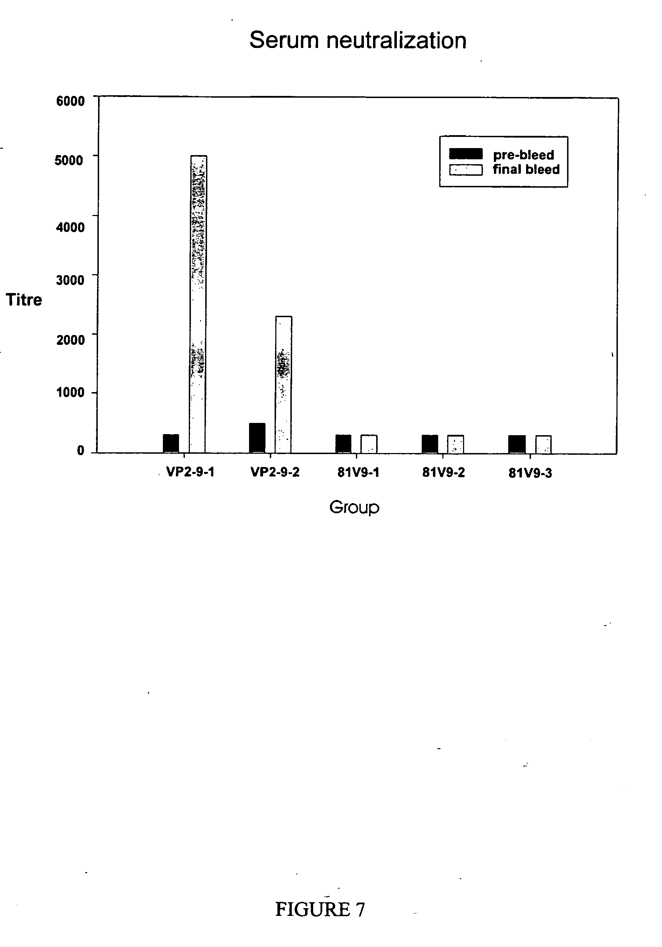

There were no differences in serum neutralization between the pre-bleed and final bleed in the control groups (Figure 7). However, in the groups injected with plant tissue containing VP2, the serum neutralization titres were considerably higher for the final bleed than for the pre-bleed. These results indicate the production of neutralizing antibodies in mice after immunization with plant produced VP2.

### Small animal trials by oral feeding

Seed collected from primary transformants is plated on MS media comprising 300 mg/L kanamycin and allowed to germinate and grow for three weeks. From the germinated seed 100 green transgenic seedlings are planted in soil-less potting mix in plug trays and placed in a greenhouse. The plants are allowed to grow for six weeks after which and the leaf material is harvested and flash frozen in liquid nitrogen. This leaf material may be used to prepare pure protein for injection or as a supplement for feeding. Transgenic leaf extracts are prepared by harvesting the leaves and rapidly freezing them in liquid nitrogen. Leaf material is mixed with a small amount of sand and round with a mortar and pestle. After the material is ground to a fine powder, two volumes of buffer (50 mM Tris buffer, 0.1 M KCL, 1mM EDTA, 5mM DTT, 15 mg/ml PVPP in 5 % glycerol) is added. The larger particles of the homogenate are removed by straining through miracloth and the solution is centrifuged to collect the virus. The pelleted virus is further purified by banding on a sucrose gradient. Following banding on a sucrose gradient, the viral band is emulsified in VSA adjuvant for intramuscular injection or mixed in PBS for oral immunization. In some oral feeding trials, the leaf material may be fed directly to mice as a pelleted chow.

The chow is prepared using transgenic or control leaf material, and is included in a diet at a level of 10 % by weight. Assuming VP2 protein concentrations of 5 *µ*g/g in the transgenic leaf material and daily chow intakes of approximately 5 g an oral dose of 25 *µ*g could be expected. The animals are fed with chow containing either transgenic or control leaf material 4 times at days 0, 4, 21 and 25. Following immunization, animals are bled on day 10, 24, and 38 by the tail vein. The levels of antibody are determined by viral neutralization and Western Blots.

### Example 6: Large Animal Trials

Piglets are immunized with different concentrations of purified VLPs by oral delivery (2 µg, 10 µg, or 50 µg/piglet) in saline. Control groups of piglets are immunized intramuscularly with equivalent concentrations of VLPs inVSA adjuvant. The animals are boosted 4 weeks after the primary dosing.

Following immunization, immune responses are monitored by assaying the level of serum antibody present at 2, 4, and 6 weeks post vaccination or boost. Antibody levels are determined using enzyme linked immuno, which are well known in the art (ELISA assays). The level of mucosal immune responses will be determined using an ELISPOT assay or ELISA for the presence of antibody in mucosal secretions.

The present invention has been described with regard to preferred embodiments. However, it will be obvious to persons skilled in the art that a number of variations and modifications can be made without departing from the scope of the invention as described herein.

### References

Arakawa, T, Ching, DKX, and Landridge, WHR (1998) Efficacy of food plant based oral cholera toxin B subunit protein. Nature Biotech. 16: 292-297.
Arntzen CJ and Lam DM (1999) Proteins expressed in plants (US 5,914,123).
Goodman RM, Knauf VC, Houck CM, Comai L (1997) Molecular Farming (US,5629,175).
Horsch, R.B., Fry, J.E., Hoffman, N.L., Eicholtz, D., Rogers, S.G., and Fraley, R.T. (1985) A si mple and general method of transferring genes into plants. Science 227:1229-1231.
Jobling SA, and Gehrke L (1987) Enhanced translation of chimaeric messenger RNAs containing a plant viral untranslated leader sequence. Nature 325:622-625.
Joo HS and Johnson RH (1976) Porcine parvovirus : a review Veterinary Bulletin 46: 653-660.
Kay R, Chan A, Daly M, and McPherson J (1987) Duplication of the 35S promoter sequences creates a strong enhancer for plant genes. Science 236:1299-1302.
Kusnadi, A.R., Nikolov, Z.L. and Howard, J.A. 1997. Production of recombinant proteins in transgenic plants: practical considerations. Biotech Bioeng. 56, 473-484.
Lam DM, Amtzen CJ and Mason HS (2000) Proteins expressed in plants (US 6,034,298).
Ma JKC, and Hein MB (1995) Immunotherapeutic potential of antibodies produced in plants TIBTECH 13: 522-527.
Mason HS, Ball JM, Shi JJ, Jiang X, Estes MK, and Amtzen CJ (1996) Expression of the Norwalk virus capsid protein in transgenic tobacco and its oral immunogenicity in plants. Proc Natl Acad Sci 93:5335-5340.
Martinez, C., Dalsgaard, K., Lopez de Turiso, J.A., Cortes, E., Vela, C., Ingnacio Casal, J. (1992) Production of porcine parvovirus empty capsids with high immunogenic activity. Protein 10:684-690.
Mengeling WL (1978) Prevalence of porcine parvovirus induced reproductive failure: an abortion study. J Amer Vet Res 37: 1393-1399.
Miki B, Hatorri J, Fobert P, Iyer V N (1998). A constitutive promoter from tobacco - US5824872.
Molitor TW Joo HS, and Collett MS (1984) Porcine parvovirus DNA: characterization of the genomic and replicative form DNA of two virus isolates. Virology 137: 241-254.
Ranz AI, Manclus JJ, Diaz-Aroca E and Casal JI (1989) Porcine parvovirus: DNA sequence and genome organization. J Gen Virol 70: 2541-2553.
Woodleif WG, Chaplin JF, Campbell CR, DeJong DW (1981) Effect of variety and harvest treatments on protein yield of close-grown tobacco. Tob Sci 25:83-86.

## Claims

1. A method of producing a protein in a plant comprising:
i) transforming said plant with a nucleotide sequence expressing a porcine parvovirus VP2 subunit protein, said nucleotide sequence comprising a nucleic acid sequence defined by SEQ ID NO:1 or a nucleotide sequence which hybridizes therewith under stringent conditions and operatively linked with a regulatory region active in said plant; and
ii) growing said transformed plant.

2. The method of claim 1 further comprising harvesting said plant and purifying said protein.

3. The method of claim 1 wherein said plant is a low nicotine tobacco plant.

4. The method of claim 1 further including extracting said VP2 subunit protein from said transformed plant and purifying or partially purifying said protein.

5. The method of claim 1 further comprising harvesting of said plant and purifying said protein for the production of a medicament.

6. An extract from a transgenic plant expressing a porcine parvovirus VP2 subunit protein, said extract comprising said VP2 subunit protein encoded by a nucleic acid sequence defined by SEQ ID NO: 1.

7. The extract of claim 6 wherein said extract when administered to an animal by injection is capable of conferring immunity against porcine parvovirus in said animal.

8. Use of the extract of claim 6 for the manufacture of a vaccine for the prevention of porcine parvovirus infection in a pig.

9. A transgenic plant, plant seed, plant cell or progeny produced therefrom comprising a nucleotide sequence expressing a porcine parvovirus VP2 subunit protein, said nucleotide sequence comprising a nucleic acid sequence defined by SEQ ID NO:1.

10. A chimeric construct comprising a regulatory region in operative association with a nucleotide sequence encoding porcine parvovirus VP2 subunit protein, said nucleotide sequence comprising a nucleic acid sequence defined by SEQ ID NO:1, and said regulatory region being active in a plant.

11. A transgenic plant, plant seed or plant cell or progeny produced therefrom comprising the chimeric construct of claim 10.

12. Use of the transgenic plant, plant seed, plant cell or progeny produced therefrom of claim 9 or 11 for the manufacture of a medicament for prevention of porcine parvovirus in a pig.

## Patentansprüche

1. Verfahren zum Herstellen eines Proteins in einer Pflanze, umfassend:
i) Transformieren der Pflanze mit einer Nukleotidsequenz, welche ein Schweine-Parvovirus-VP2-Untereinheit-Protein exprimiert, wobei die Nukleotidsequenz eine Nukleinsäuresequenz, die durch SEQ ID NR: 1 definiert wird, oder eine Nukleotidsequenz, die unter stringenten Bedingungen damit hybridisiert, umfasst und funktionsfähig (operativ) mit einer regulatorischen Region, die in der Pflanze aktiv ist, verknüpft ist; und
ii) Kultivieren der transformierten Pflanze.

2. Verfahren nach Anspruch 1, welches des Weiteren das Ernten der Pflanze und das Reinigen des Proteins umfasst.

3. Verfahren nach Anspruch 1, wobei die Pflanze eine Tabakpflanze mit niedrigem Nicotingehalt ist.

4. Verfahren nach Anspruch 1, welches des Weiteren das Extrahieren des VP2-Untereinheit-Proteins aus der transformierten Pflanze und das Reinigen oder teilweise Reinigen des Proteins umfasst.

5. Verfahren nach Anspruch 1, welches des Weiteren das Ernten der Pflanze und das Reinigen des Proteins für die Herstellung eines Arzneimittels umfasst.

6. Extrakt aus einer transgenen Pflanze, welche ein Schweine-Parvovirus-VP2-Untereinheit-Protein exprimiert, wobei der Extrakt das VP2-Untereinheit-Protein, welches durch eine durch SEQ ID NR: 1 definierte Nukleinsäuresequenz kodiert wird, umfasst.

7. Extrakt nach Anspruch 6, wobei der Extrakt, wenn er einem Tier durch Injektion verabreicht wird, in der Lage ist, Immunität gegen Schweine-Parvoviren in dem Tier zu verleihen.

8. Verwendung des Extrakts nach Anspruch 6 für die Herstellung eines Impfstoffs für die Verhütung einer Infektion durch Schweine-Parvoviren bei einem Schwein.

9. Transgene Pflanze, transgener Pflanzensamen, transgene Pflanzenzelle oder ausgehend davon hergestellte Nachkommenschaft, umfassend eine Nukleotidsequenz, welche ein Schweine-Parvovirus-VP2-Untereinheit-Protein exprimiert, wobei die Nukleotidsequenz eine durch SEQ ID NR: 1 definierte Nukleinsäuresequenz umfasst.

10. Chimäres Konstrukt, welches eine regulatorische Region in funktionsfähiger Assoziation mit einer Nukleotidsequenz, welche ein Schweine-Parvovirus-VP2-Untereinheit-Protein kodiert, umfasst, wobei die Nukleotidsequenz eine durch SEQ ID NR: 1 definierte Nukleinsäuresequenz umfasst und die regulatorische Region in einer Pflanze aktiv ist.

11. Transgene Pflanze, transgener Pflanzensamen oder transgene Pflanzenzelle oder ausgehend davon hergestellte Nachkommenschaft, umfassend das chimäre Konstrukt nach Anspruch 10.

12. Verwendung der transgenen Pflanze, des transgenen Pflanzensamens, der transgenen Pflanzenzelle oder von ausgehend davon hergestellter Nachkommenschaft nach Anspruch 9 oder 11 für die Herstellung eines Arzneimittels für die Verhütung von Schweine-Parvoviren bei einem Schwein.

## Revendications

1. Une méthode de production d'une protéine par une plante comprenant :
i) la transformation de ladite plante à l'aide d'une séquence nucléotidique comprenant une protéine d'une sous-unité VP2 du parvovirus porcin, ladite séquence nucléotidique comprenant une séquence nucléotidique telle que définie par SEQ ID N° 1 ou une séquence nucléotidique qui s'hybride avec celle-ci sous des conditions stringentes et liée opérativement avec une région régulatrice active dans ladite plante ; et
ii) la culture de ladite plante transformée.

2. La méthode selon la revendication 1, comprenant en outre la récolte de ladite plante et la purification de ladite protéine.

3. La méthode selon la revendication 1, dans laquelle ladite plante est un plant de tabac à faible teneur en nicotine.

4. La méthode selon la revendication 1, comprenant en outre l'extraction de ladite protéine d'une sous-unité VP2 de ladite plante transformée et la purification ou purification partielle de ladite protéine.

5. La méthode selon la revendication 1, comprenant en outre la récolte de ladite plante et la purification de ladite protéine pour la production d'un médicament.

6. Un extrait d'une plante transgénique exprimant une protéine d'une sous-unité VP2 de parvovirus porcin, ledit extrait comprenant ladite sous-unité protéique VP2 codée par une séquence nucléotidique telle que définie par SEQ ID N° 1.

7. L'extrait selon la revendication 6, dans laquelle ledit extrait, administré à un animal par injection, est capable de conférer une immunité, vis-à-vis du parvovirus porcin chez ledit animal.

8. L'utilisation de l'extrait selon la revendication 6 pour la fabrication d'un vaccin pour la prévention d'une infection à parvovirus porcin chez le cochon.

9. Une plante, graine de plante, cellule de plante ou progénique ou leur descendance obtenue à partir de ceux-ci comprenant une séquence nucléotidique exprimant une protéine d'une sous-unité VP2 de parvovirus porcin, ladite séquence nucléotidique comprenant une séquence d'acide nucléique telle que définie par SEQ ID N° 1.

10. Une construction chimérique comprenant une région régulatrice en association opérative avec une séquence nucléotidique codant pour la protéine d'une sous-unité VP2 de parvovirus porcin, ladite séquence nucléotidique comprenant une séquence d'acide nucléique telle que définie par SEQ ID n° 1, et ladite région régulatrice étant active chez une plante.

11. Une plante, graine de plante ou cellule de plante transgénique ou leur descendance comprenant la construction chimérique selon la revendication 10.

12. Utilisation de la plante, graine de plante, cellule de plante transgénique ou leur descendance obtenue à partir de ceux-ci selon l'une quelconque des revendications 9 ou 11 pour la fabrication d'un médicament pour la prévention du parvovirus porcin chez le cochon.


### SEQUENCE LISTING

<110> Her Majesty the Queen in Right of Canada, as Represented by the Minister of Agriculture and Agrifoo Canada
   Veterinary Infectious Disease Organization
   Rymerson, Robert
   Brandle, Jim
   Babiuk, Lorne
<120> Recombinant Subunit Proteins from Porcine Parvovirus Produced in Plants
<130> 087-887541WO
<150> 60/208,941
   <151> 2000-06-05
<160> 4
<170> PatentIn version 3.0
<210> 1
   <211> 1802
   <212> DNA
   <213> synthetic VP2subunit protein
<400> 1
   ggatcccccc tagagttttt atttttaatt tttctttcaa atacttccat catgagtgaa      60
   aatgtggaac aacacaaccc tattaatgca ggcactgaat tgtctgcaac aggaaatgaa     120
   tctgggggtg ggggcggcgg tggcgggggt aggggtgctg ggggggttgg tgtgtctaca     180
   ggtagtttca ataatcaaac agaatttcaa tacttggggg agggcttggt tagaatcact     240
   gcacacgcat caagactcat acatctaaat atgccagaac acgaaacata caaaagaata     300
   catgtactaa attcagaatc aggggtggcg ggacaaatgg tacaagacga tgcacacaca     360
   caaatggtaa caccttggtc actaatagat gctaacgcat ggggggtgtg gttcaatcca      420
   gcggactggc agttaatatc caacaacatg acagaaataa acttagttag ttttgaacaa     480
   gaaatattca atgtagtact taaaacaatt acagaatcag caacctcacc accaaccaaa     540
   atatataata atgatctaac tgcaagctta atggtcgcac tagacaccaa taacacactt     600
   ccatacacac cagcagcacc tagaagcgaa acacttggtt tttacccatg gttacctaca     660
   aaaccaactc aatacagata ttacctatca tgcaccagaa acctaaatcc accaacatac     720
   actggacaat cacaacaaat aacagactca atacaaacag gactacacag tgacattatg     780
   ttctacacaa tagaaaatgc agtaccaatt catcttctaa gaacaggaga tgaattctcc     840
   acaggaatat atcactttga cacaaaacca ctaaaattaa ctcactcatg gcaaacaaac     900
   agatctctag gactgcctcc aaaactacta actgaaccta ccacagaagg agaccaacac     960
   ccaggaacac taccagcagc taacacaaga aaaggttatc accaaacaat taataatagc    1020
   tacacagaag caacagcaat taggccagct caggtaggat ataatacacc atacatgaat    1080
   tttgaatact ccaatggtgg accatttcta actcctatag taccaacagc agacacacaa    1140
   tataatgatg atgaaccaaa tggtgctata agatttacaa tgggttacca acatggacaa    1200
   ttaaccacat cttcacaaga gctggaaaga tacacattca atccacaaag taaatgtgga    1260
   agagctccaa agcaacaatt taatcaacag gcaccactaa acctagaaaa tacaaataat    1320
   ggaacacttt taccttcaga tccaatagga gggaaaccta acatgcattt catgaataca    1380
   ctcaatacat atggaccatt aacagcacta aacaatactg cacctgtatt tccaaatggc    1440
   caaatatggg ataaagaact tgatacagat ctaaaaccta gactacatgt tacagctcca    1500
   tttgttcgta aaaacaatcc accaggacaa ctatttgtaa aaatagcacc aaacctaaca    1560
   gatgatttca atgctgactc tcctcaacaa cctagaataa taacttattc aaacttttgg    1620
   tggaaaggaa cactaacatt cacagcaaaa atgagatcca gtaatatgtg gaaccctatt    1680
   caacaacaca caacaacagc agaaaacatt ggtaactata ttcctacaaa tattggtggc    1740
   ataaaaatgt ttccagaata ttcacaactt ataccaagaa aattatacta gtaataactc    1800
   ta                                                                    1802
<210> 2
   <211> 70
   <212> DNA
   <213> synthetic AMWP primer
<400> 2
   gggtctagag tttttatttt taatttttct ttcaaatact tccatcatga gtgaaaatgt 60
   ggaacaacac 70
<210> 3
   <211> 19
   <212> DNA
   <213> synthetic VP1060F
<400> 3 19
   ggtggaccat ttctaactc 19
<210> 4
   <211> 23
   <212> DNA
   <213> synthetic nos primer
<400> 4 23
   ccggcaacag gattcaatct taa 23